# FASCICULE DE BREVET EUROPEEN

(11) **EP 0 348 252 B1**
(45) Date de publication et mention de la délivrance du brevet: **28.07.1993**
(21) Numéro de dépôt: 89401418.2
(22) Date de dépôt: 24.05.1989
(51) Int. Cl.: B29C 59/16, A61F 2/30, A61L 27/00, C08J 7/00

(54) **Procédé pour réduire le coefficient de frottement et l'usure entre une piéce métallique et une pièce à base d'un polymère ou copolymère organique et son application à des prothèses articulaires et des emboîtements travaillant dans des milieux marins**
Verfahren zum Erniedrigen des Reibungskoeffizienten und des Verschleisses zwischen einem metallischen Teil und einem Teil aus einem organischen Polymer oder Copolymer und dessen Anwendung bei Gelenkprothesen und im maritimen Bereich wirkenden Gelenkverbindungen
Method for reducing the friction coefficient and the wear between a metallic part and a part consisting of an organic polymer or copolymer, and its use in articular prostheses and to socket joints employed in marine environments

(30) Priorité: 24.05.1988 FR 8806890
(43) Date de publication de la demande: 27.12.1989
(73) Titulaire: NITRUVID, 95100 Argenteuil (FR)
(72) Inventeur: Chabrol, Claude, F-69700 Givors (FR); Robelet, Marc, F-42240 Unieux (FR); Leveque, Robert, F-42700 Firminy (FR); Pichat, Anne, Louise, Marie (née Nedelec), F-42100 St. Etienne (FR); Rieu, Jean-Francois, Emile,, F-42100 St. Etienne (FR); Rabbe, Louis Marie,, F-42490 Fraisses (FR); Rambert, André, F-69003 Lyon (FR)
(74) Mandataire: Le Guen, Gérard

(56) Documents cités:
- FR-A- 2 478 113
- US-A- 4 743 493
- APPLIED OPTICS, vol. 19, no. 17, premier September 1980, pages 3022-3026, Optical Society of America, New York, US; E. SPILLER et al.: "Graded-index AR surfaces produced by ion implantation on plastic materials"
- CHEMICAL ABSTRACTS, vol. 104, no. 12, 24 mars 1986, page 442, résumé no. 95419w, Columbus, Ohio, US; F. MATTHEWS et al.: "Enhancement of the TI-6AL-4V/UHMWPE wear couple through nitrogen ion implantation", & BIOMED. ENG. RECENT DEV., PROC. SOUTH. BIOMED. ENG. CONF. 4TH, 1985, p18-21

## Description

La présente invention concerne un procédé pour réduire le coefficient de frottement et l'usure entre une pièce métallique et une pièce à base d'un polymère ou copolymère organique et trouve une application dans des prothèses articulaires et dans des emboitements travaillant dans des milieux marins.

Les prothèses articulaires ou les emboitements à rotation ou à glissement comportent généralement deux pièces qui frottent l'une contre l'autre. Généralement, au moins l'une de ces pièces est métallique. L'autre est en polymère ou est recouverte d'une couche à base d'un polymère ou copolymère organique. La résistance au frottement et à l'usure du couple est acceptable mais pourrait être améliorée par des traitements de surfaces adaptés.

On utilise généralement comme polymère du polyéthylène. Les métaux utilisés sont généralement des aciers inoxydables, des alliages à base de cobalt ou de titane. A la place des métaux, on peut utiliser également d'autres matériaux (céramiques, composites comme le carbone-carbone, etc.).

La présente invention vise à fournir un procédé permettant notamment de réduire le frottement et l'usure entre de telles pièces fonctionnant dans un environnement aqueux chloruré tel que le liquide physiologique ou le milieu marin.

A cet effet, la présente invention a pour objet un procédé pour réduire le coefficient de frottement et l'usure entre une pièce métallique et une pièce à base d'un polymère ou copolymère organique, dans un milieu aqueux chloruré, caractérisé en ce que l on fait subir à la pièce à base d'un polymère ou copolymère organique, un traitement superficiel par implantation ionique, d'au moins un élément choisi parmi l'azote, l'argon, l'oxygenè et le carbone.

La présente invention s'applique en particulier dans le cas où le polymère est un polyéthylène, tel qu'un polyéthylène haute densité, et à très haut poids moléculaire, mais d'autres polymères peuvent être utilisés : polymères basse densité, polymères renforcés (fibres, particules..), etc..

Le principe de l'implantation ionique consiste à ioniser au moins une espèce atomique choisie parmi l'azote, l'argon, l'oxygène, le carbone ou autre, puis à l'accélérer dans un champ électrique à des énergies de 10 à 500 keV, de préférence de 10 à 300 keV et plus avantageusement encore de 50 à 150 keV.

On opère généralement sous des pressions de 10⁻² à 10⁻⁴ Pa. Dans ces conditions, les ions pénètrent dans la couche à base de polymère ou de copolymère jusqu'à une profondeur qui peut atteindre plusieurs microns.

La dose d'implantation est généralement de 10¹² à 10¹⁷ ions/cm².

Le compte rendu des essais suivants met en évidence l'amélioration obtenue par la mise en oeuvre du procédé selon l'invention.

### 1) Implantation ionique de polyéthylène haute densité et à très haut poids moléculaire.

Le polyéthylène utilisé est de l'Ertalène HD 1000, CESTILENE MC (densité 0,94, module d'élasticité de 650 MPa), conforme aux normes ISO 5834/2 et NF S90 - 418 et/ou du polyéthylène à poids moléculaire élevé.

Des échantillons cylindriques (pions) et hémi-sphériques (cupules) de polyéthylène ont subi un traitement d'implantation ionique d'azote, d'argon, d'oxygène, de carbone et autres, dans un appareil mis au point et fabriqué par UNIREC. (Robert LEVEQUE et Claude CHABROL, Application de l'implantation ionique dans le domaine de la construction mécanique, Journées de GAMI les 8 et 9 mars 1988, Paris).

L'énergie d'implantation a été de 80 keV.

### 2) Essais

Des essais de frottement entre des pièces en polyéthylène et des pièces métalliques (acier inoxydable conforme aux normes ISO 5832-I-NF S90-401 et alliage de titane conforme aux normes ISO 5832-III-NF S90-405 sur deux machines mises au point et fabriquées à l'Ecole Nationale Supérieure des Mines de Saint-Etienne.

L'une de ces machines est du type pion-disque, l'autre est du type sphère-cupule. (A. PICHAT, J. RIEU, C.CHABROL, A.RAMBERT, Effet de l'implantation ionique sur la résistance à l'usure et à la fatique de matériaux pour applications orthopédiques. Colloque Int. sur les matériaux résistant à l'usure, Bulletin du Cercle d'Etude des Métaux, 1987, 15-14, p. 9-1).

Les essais sont réalisés sur des pièces de qualité orthopédique fabriquées par la société SERF, à Décines.

Les conditions d'essais (contrainte, vitesse, environnement sont proches de celles d'une prothèse de hanche).

Tous les essais sont réalisés en milieu liquide physiologique artificiel (solution de RINGER).

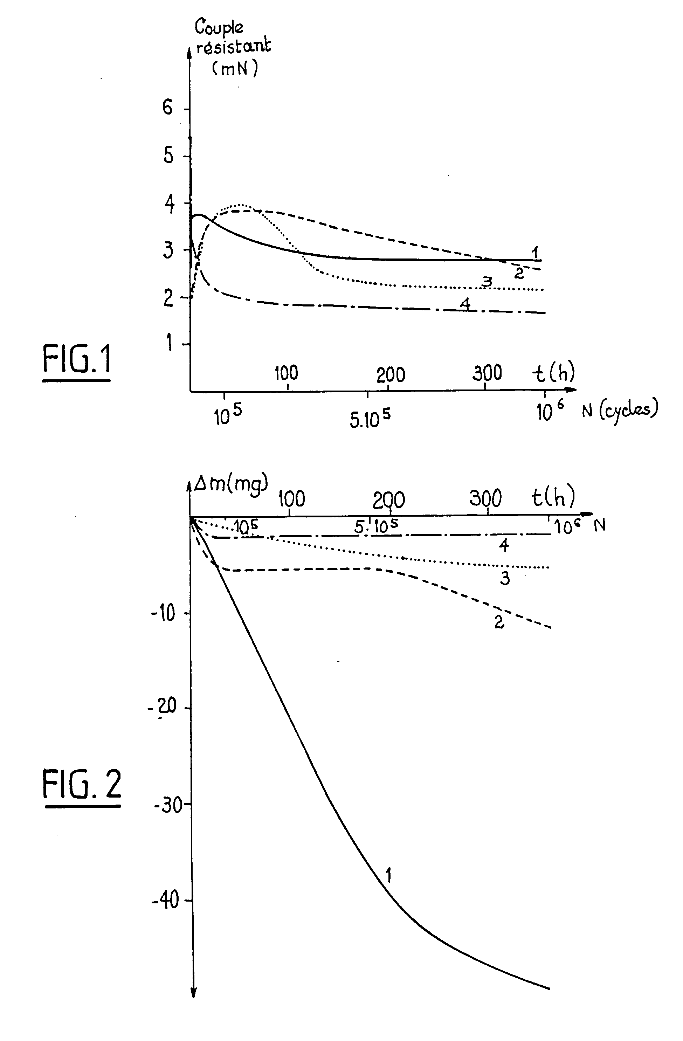

A titre d'exemple, les résultats obtenus sur le couple sphère en acier inoxydable - cupule en polyéthylène, sont reportés sur les figures 1, 2, 3 et 4.

Les courbes montrent l'évolution du couple résistant (Fig.1) et celle du taux d'usure du polyéthylène exprimé en perte de mase m (Fig.2), en fonction du temps t et du nombre de cycles N avec :
1 - couple PE non traité / Acier inoxydable 316L non traité
2 - couple PE non traité / Acier inoxydable 316L implanté à l'azote
3 - couple PE implanté à l'azote/ Acier inoxydable 316L non traité
4 - couple PE implanté à l'azote/ Acier inoxydable 316L implanté à l'azote.

Ces essais ont été arrêtés à 1 million de cycles; des essais plus longs ont toutefois permis de vérifier que les effets bénéfiques de l'implantation étaient conservés.

Ces résultats mettent en évidence qu'en l'absence de toute implantantion, l'usure est importante et qu'en pratique l'implantation du polyéthylène seule suffit déjà pour obtenir un couple de frottement et un taux d'usure du polyéthylène notablement plus faibles (courbe 3).

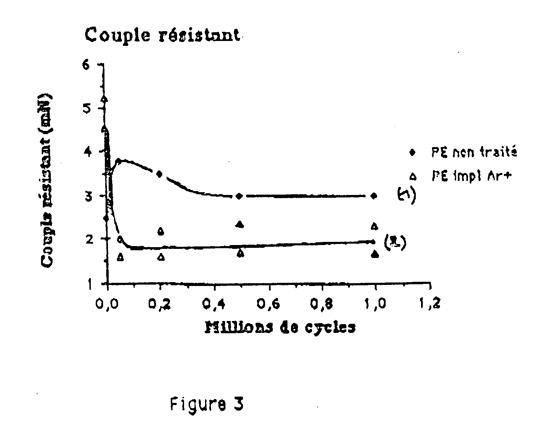

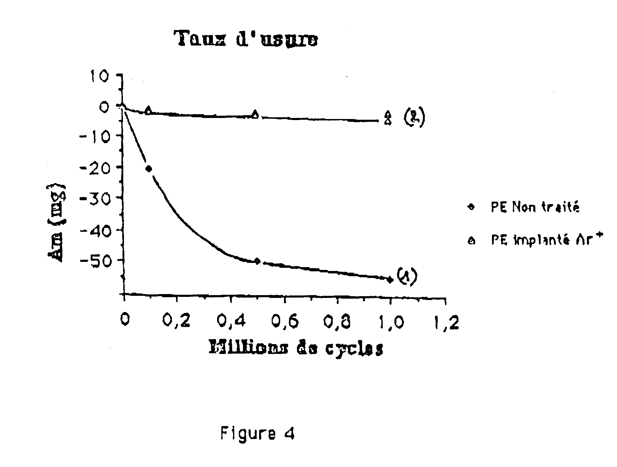

Les Figures 3 et 4 sont relatives aux mêmes essais de sphères en acier inoxydable 316 L non traité en comparaison avec des cupules en polyéthylène non traité ou traité par implantation d'ions argon. La Fig.3 montre que le couple résistant est plus faible lorsque le polyéthylène est implanté à l'argon (courbe 2) que lorsqu'il n'est pas traité (courbe 1, identique à la courbe 1 de la Fig.1).

La Fig.4 montre que le taux d'usure Δ m du polyéthylène est beaucoup plus faible (5 mg contre 50 mg) lorsque le polyéthylène est implanté à l'argon (courbe 2) que lorsqu'il n'est pas traité (courbe 1, identique à la courbe 1 de la Fig.2).

Ces résultats montrent qu'une implantation ionique du polyéthylène avec de l'argon donne une diminution du couple de frottement et une réduction du taux d'usure du polyéthylène comparables à celles obtenues avec une implantation ionique du polyéthylène avec de l'azote.

Lors des essais d'implantation ionique d'argon et d'azote, il est introduit de manière incontrôlée, une faible quantité de carbone qui contribue aux résultats des essais décrits ci-dessus.

## Revendications

1. Procédé pour réduire le coefficient de frottement et l usure entre une pièce métallique et une pièce à base d'un polymère ou copolymère organique, dans un milieu aqueux chloruré, caractérisé en ce que l'on fait subir à la pièce à base d'un polymère ou copolymère organique, un traitement superficiel par implantation ionique d'au moins un élément choisi parmi l'azote, l'argon, l'oxygène et le carbone.

2. Procédé selon la revendication 1, caractérisé en ce que le polymère est un polyéthylène.

3. Procédé selon l'une quelconque des revendications 1 ou 2, caractérisé en ce que l'implantation est effectuée avec une énergie de 10 à 300 keV.

4. Procédé selon la revendication 3, caractérisé en ce que l'implantation est effectuée avec une énergie de 50 à 150 keV.

5. Elément de prothèse articulaire du type comportant une pièce métallique frottant sur une pièce ou une couche d'un polymère ou copolymère organique, caractérisée en ce que la surface de l'élément de polymère organique en contact avec la pièce métallique a été traitée par un procédé selon l'une quelconque des revendications 1 à 4.

6. Prothèse articulaire comportant un élément tel que défini à la revendication 5.

7. Emboitements à rotation ou à glissement travaillant dans des milieux marins et comportant une pièce métallique frottant sur une pièce ou une couche d'un polymère ou copolymère organique, caractérisés en ce que la surface de l'élément de polymère organique en contact avec la pièce métallique a été traité par un procédé selon l'une quelconque des revendications 1 à 4.

## Claims

1. A method of reducing the coefficient of friction and the wear between a metal part and a part with an organic polymer or copolymer basis, in a chlorinated aqueous medium, **characterised in that** the part with an organic polymer or copolymer basis is subjected to surface treatment by ion implantation of at least one element selected from nitrogen, argon, oxygen and carbon.

2. A method according to claim 1, **characterised in that** the polymer is a polyethylene.

3. A method according to either of claims 1 or 2, **characterised in that** the implantation is carried out with an energy of from 10 to 300 keV.

4. A method according to claim 3, **characterised in that** the implantation is carried out with an energy of from 50 to 150 keV.

5. A component of an artificial limb joint of a type comprising a metal part sliding against a part or a layer of an organic polymer or copolymer, **characterised in that** the surface of the organic polymer component in contact with the metal part has been treated using a method according to any one of claims 1 to 4.

6. An artificial limb joint comprising a component as defined in claim 5.

7. Rotating or sliding joints operating in marine environments and comprising a metal part sliding against a part or a layer of an organic polymer or copolymer, **characterised in that** the surface of the organic polymer component in contact with the metal part has been treated using a method according to any one of claims 1 to 4.

## Patentansprüche

1. Verfahren zur Verringerung des Reibungskoeffizienten und des Abriebs zwischen einem Metallteil und einem Teil auf der Grundlage eines organischen Polymers oder Copolymers in einem chloridhaltigen wäßrigen Medium, dadurch **gekennzeichnet,** daß das Teil auf der Grundlage eines organischen Polymers oder Copolymers einer Oberflächenbehandlung durch Ionenimplantation unterworfen wird, mit Ionen, die aus mindestens einem unter Stickstoff, Argon, Sauerstoff und Kohlenstoff ausgewählten Element bestehen.

2. Verfahren nach Anspruch 1, dadurch gekennzeichnet, daß das Polymer ein Polyethylen ist.

3. Verfahren nach einem der Ansprüche 1 oder 2, dadurch gekennzeichnet, daß die Implantation mit einer Energie von 10 bis 300 keV durchgeführt wird.

4. Verfahren nach Anspruch 3, dadurch gekennzeichnet, daß die Implantation mit einer Energie von 50 bis 150 keV durchgeführt wird.

5. Gelenkprothesenelement vom Typ mit einem Metallteil, das auf einem Teil oder einer Schicht aus einem organischen Polymer oder Copolymer reibt, dadurch gekennzeichnet, daß die Oberfläche des organischen Polymerelements, die das Metallteil berührt, in einem Verfahren nach einem der Ansprüche 1 bis 4 behandelt worden ist.

6. Gelenkprothese mit einem Element nach Anspruch 5.

7. Dreh- oder Gleitverbindungen zur Anwendung in Seewassermedien mit einem Metallteil, das auf einer Schicht oder einem Teil aus einem organischen Polymer oder Copolymer reibt, dadurch gekennzeichnet, daß die Oberfläche des Elements aus organischem Polymer, die das Metallteil berührt, in einem Verfahren nach einem der Ansprüche 1 bis 4 behandelt worden ist.
